(19) Europäisches Patentamt
European Patent Office
Office européen des brevets

(11) EP 0 642 527 B1

(12) # EUROPÄISCHE PATENTSCHRIFT

(45) Veröffentlichungstag und Bekanntmachung des Hinweises auf die Patenterteilung:
**10.01.1996 Patentblatt 1996/02**

(21) Anmeldenummer: **93905240.3**

(22) Anmeldetag: **12.02.1993**

(51) Int Cl.$^6$: **C07H 19/10**, A61K 31/70

(86) Internationale Anmeldenummer:
**PCT/EP93/00346**

(87) Internationale Veröffentlichungsnummer:
**WO 93/16093 (19.08.1993 Gazette 1993/20)**

(54) **AMPHIPHILE NUCLEOSIDPHOSPHATANALOGA**
AMPHIPHILIC NUCLEOSIDE PHOSPHATE ANALOGS
ANALOGUES DE PHOSPHATES NUCLEOSIDES AMPHIPHILES

(84) Benannte Vertragsstaaten:
**AT CH DE DK ES FR GB IT LI NL SE**

(30) Priorität: **13.02.1992 DE 4204211**
**28.07.1992 DE 4224878**

(43) Veröffentlichungstag der Anmeldung:
**15.03.1995 Patentblatt 1995/11**

(73) Patentinhaber: **SCHOTT, Herbert**
**D-72076 Tübingen (DE)**

(72) Erfinder:
- **SCHWENDENER, Albert, Reto**
**CH-8802 Kilchberg (CH)**
- **GUERIN, Frédérique**
**F-75014 Paris (FR)**
- **Schott, Herbert Prof. Dr.**
**D-72016 Tübingen (DE)**

(74) Vertreter: **Kinzebach, Werner, Dr. et al**
**D-81633 München (DE)**

(56) Entgegenhaltungen:
**EP-A- 0 392 791** **WO-A-90/06319**

- **JOURNAL OF MEDICINAL CHEMISTRY Bd. 31, 1988, WASHINGTON US Seiten 2040 - 2048 P.HERDEWIJN ET AL. 'Synthesis and Anti-HIV Activity of Different Sugar-Modified Pyrimidine and Purine Nucleosides.'**
- **PROCEEDINGS OF THE NATIONAL ACADEMY OF SCIENCES OF USA. Bd. 83, Nr. 21,**
- **November 1986, WASHINGTON US Seiten 8333 - 8337 P.A.FURMAN ET AL. 'Phosphorylation of 3'-Azido-3'-Deoxythymidine and Selective Interaction of the 5'-Triphosphate with Human Immunodeficiency Virus Reverse Transcriptase.' in der Anmeldung erw hnt**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Die vorliegende Erfindung betrifft neue amphiphile Nucleosidphosphatanaloga, deren Herstellung sowie Mittel zur Behandlung von Krebserkrankungen und Infektionskrankeiten.

Nucleosidanaloga, die bestimmte Strukturmerkmale aufweisen, sind bewährte Arzneimittel in der Chemotherapie von Krebserkrankungen und von virusbedingten Infektionskrankheiten (AIDS Res. Human Retroviruses 8, 119 (1992)). Die therapeutische Wirkung von Cytosinnucleosidanaloga beispielsweise von araC ist dadurch limitiert, daß körpereigene Cytosindesaminasen die Aminogruppe der Nucleobase zu schnell desaminieren, und die dabei gebildeten Uracilnucleosidanaloga in der Regel therapeutisch unwirksam sind (Biochem. Pharmacol. 33, 2159 (1984)). Zur Erzielung einer therapeutischen Wirkung müssen Cytosinnucleosidanaloga daher in hohen Dosen appliziert werden, die für den Patienten mit schwerwiegenden Nebenwirkungen verbunden sind. Mit Cytosinnucleosidanaloga, deren Aminogruppen mit Schutzgruppen versehen sind, versucht man die therapeutische Wirkung zu optimieren.

3'-Azido-2',3'-didesoxythymidin (AZT) und 2',3'-Didesoxycytidin (ddC) sind beispielsweise in der Chemotherapie der AIDS-Erkrankung die am häufigsten angewendeten Virusstatika. Die antiretrovirale Wirkung basiert darauf, daß AZT und ddC nach der Zellaufnahme zum 5'-Triphosphatderivat anabolisiert werden, die dann selektiv die Reverse-Transkriptase des Humanen-Immundefizienz Virus (HIV) binden, durch das die AIDS-Erkrankung ausgelöst wird. (Proc. Natl. Acad. Sci. U.S.A. 1986, 38, 8333 - 8337). Mit AZT-Derivaten, die von den infizierten Zellen besser aufgenommen werden oder aufgrund einer behinderten Hydrolyse AZT nur verzögert freisetzen (Depotwirkung), versucht man, die Anti-HIV-Wirkung von AZT zu steigern und die schwerwiegenden toxischen Nebenwirkungen zu mindern. Nach einem anderen Konzept versucht man mit Liposomen, die AZT verkapselt oder Glycerophospholipid-AZT-Konjugate in ihrer Lipidmembran eingebaut haben, das große HIV-Reservoir von Makrophagen gezielt auszuschalten. Man hofft, daß die Makrophagen den größten Teil der applizierten Liposomen bevor sie hydrolysieren phagozitieren, so daß nur wenig freigesetztes AZT von anderen Zellen aufgenommen wird und damit die toxischen Nebenwirkungen verringert werden. In einem neuen Therapieschema, mit dem der therapeutische Index von AZT verbessert werden kann, wird AZT zusammen mit 2', 3'-Didesoxycytidin (ddC) appliziert. Den Vorteil einer solchen Kombinationstherapie versucht man auch über Dinucleosidphosphatanaloga zu erreichen, in denen AZT mit anderen antiretroviralen Didesoxynucleosidanaloga kovalent verbunden ist, wobei allerdings die wirksamsten Nucleoside AZT und ddC noch nicht gekuppelt wurden (AIDS Res. Human. Retro. 1988, 4, 449 - 454; Antimicrob. Agents Chemother. 1990, 34, 1061 - 1067). Die Dimerisierung unterschiedlicher antiretroviral wirksamer Verbindungen ist allerdings nur dann zweckmäßig, wenn beide Reagenzien bei etwa der gleichen Konzentration ihre Wirkung entfalten. Ein bedenklicher Nachteil der beschriebenen Dimeren ist ihr zu hydrophiler Charakter. Hierdurch werden die Passage durch die Zellmembran und der stabile Einbau in Liposomen erschwert, sowie die enzymatische Hydrolyse nicht verzögert.

Die WO 90/06319 offenbart unter anderem antivirale Dinucleotide der Formel:

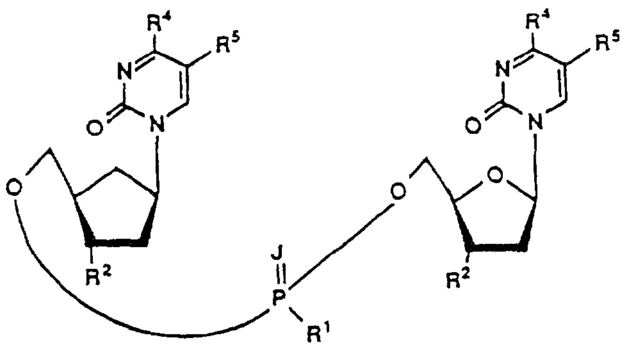

worin

J für 0 oder S steht;

$R^1$ für OH, $OCH_3$, $CH_3$ oder OB steht, worin B für bestimmte 5-gliedrige Ringe stehen kann;

$R^2$ für H oder $N_3$ steht;

$R^4$ jeweils für OH oder $NH_2$ stehen kann; und

$R^5$ jeweils für H, $CH_3$ oder ein Halogenatom stehen kann.

Aus der EP-A- 0 392 791 sind antivirale Substanzen der Formel

$$R_1\ X\ ArO\ \overset{\overset{O}{\|}}{P}\ (OR_2)(OR_3)$$

bekannt, worin

$R_1$ für eine aliphatische Hydrocarbylgruppe steht,

Ar für einen substituierten oder unsubstituierten aromatischen Rest steht;

X für $-SO_2-$ oder -CO- steht; und

$R_2$ und $R_3$ für bestimmte Nucleosidreste stehen, die im Vergleich zu üblichen Nucleosiden am Riboserest modifiziert sind.

Aufgabe dieser Erfindung ist es, neue Nucleosidphosphatanaloga bereitzustellen, mit denen Krebserkrankungen und Infektionen noch wirksamer bekämpft werden können.

Die Aufgabe wurde durch neue amphiphile Nucleosidphosphatanaloga gelöst, welche die Vorteile der genannten unterschiedlichen Konzepte vereinigen und die mit einem vertretbaren Aufwand in präparativen Mengen zugänglich sind. Hierbei wurden lipophile Nucleotidderivate an hydrophile therapeutisch wirksame Nucleosidanaloge über Phosphodiesterbrücken kovalent verbunden oder lipophile therapeutisch wirksame Nucleosidanaloga an hydrophile Nucleotidderivate gekuppelt.

Gegenstand der Erfindung sind somit amphiphile Nucleosidphosphatanaloga der Formel I

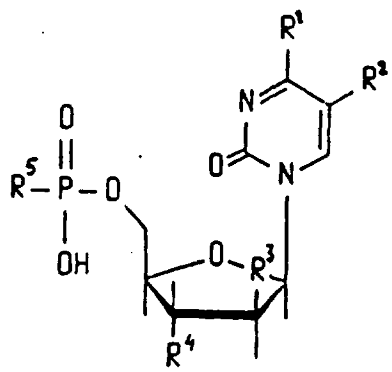

I,

worin

$R^1$ eine Hydroxyl-, Amino-, acylierte oder alkylierte oder durch Polyoxyethylen substituierte Aminogruppe bedeutet, deren Acyl- oder Alkylrest linear oder verzweigt ist, 1 bis 24 C-Atome und bis zu 2 Doppelbindungen aufweist und durch einen aromatischen Rest substituiert sein kann;
$R^2$ für H, F, einen 2-Bromvinylrest oder einen linearen oder verzweigten $C_1$-$C_{24}$-Alkylrest steht;
$R^3$ und $R^4$ gleich oder verschieden sind und für H, Hydroxyl, Halogen und Azido stehen;
$R^5$ einen Nucleosidrest der Formel II

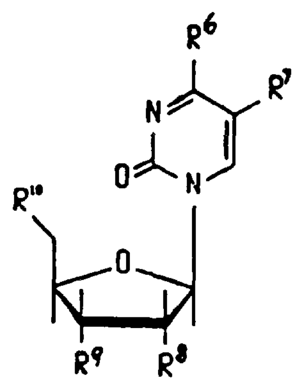

II,

bedeutet, worin

$R^6$ eine Hydroxyl-, Amino-, acylierte oder alkylierte Aminogruppe bedeutet, deren Acyl- oder Alkylrest linear oder verzweigt ist, 1 bis 24 C-Atome und bis zu 2 Doppelbindungen aufweist und durch einen aromatischen Rest substituiert sein kann,
wobei

$R^6$ und $R^1$ verschieden sein sollen;

$R^7$ für H, F, einen 2-Bromvinylrest oder einen linearen oder verzweigten $C_1$ bis $C_{24}$-Alkylrest steht;
einer der Reste $R^8$ bis $R^{10}$ ein Sauerstoffatom bedeutet, das die Brücke zum Nucleotid der Formel I bildet, die beiden verbleibenden Reste gleich oder verschieden sind und für H, Hydroxyl, Halogen oder Azido stehen;

$R^5$ auch OH bedeuten kann,
sowie die entsprechenden Salze der sauren Formen dieser Verbindungen.

Ist $R^1$ oder $R^6$ eine alkylierte Aminogruppe, so ist deren Alkylrest vorzugsweise ein Hexadecyl- oder Oktadecylrest.

Ist $R^1$ oder $R^6$ eine acylierte Aminogruppe, so ist deren Acylrest bevorzugt ein Palmitoyl-, Oleoyl- und Behenoyl-(Docosanoyl)-Rest.

Für $R^2$ sind Wasserstoff, Fluor, Methyl, Ethyl; für $R^3$, $R^4$, $R^8$ Wasserstoff oder eine Hydroxylgruppe; für $R^7$ Wasserstoff oder eine Methylgruppe und für $R^9$ und $R^{10}$ sind Wasserstoff, Sauerstoff oder eine Hydroxylgruppe bevorzugt.

Als Halogensubstituenten sind F, Cl und Br bevorzugt. Wenn $R^1$ oder $R^6$ im Acyl- oder Alkylrest aromatisch substituiert sind, so ist dies vorzugsweise ein Phenylsubstituent.

Wie oben bereits angedeutet, sollen erfindungsgemäß die Nucleosidreste so hydrophil oder lipophil sein, daß das Dinucleosidphosphatanaloga amphiphile Eigenschaften hat. Daher stellen $R^1$ und /oder $R^2$ lipophile Reste und $R^6$ und/oder $R^7$ hydrophile Reste dar oder vice versa oder die Reste $R^1$, $R^2$, $R^6$ und $R^7$ werden so gewählt, daß sie gemeinsam dem Dinucleosidphosphatanalog amphiphilen Charakter verleihen.

Die neuen amphiphilen Dinucleosidphosphatanaloga der Formel I lassen sich herstellen, indem man Verbindungen der Formel III

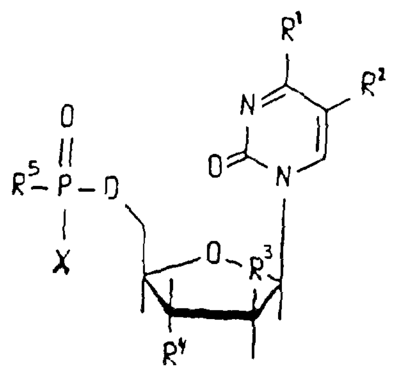

III,

worin $R^1$ bis $R^5$ die angegebenen Bedeutungen haben, $R^5$ aber nicht für OH steht, und
X den Chlorphenoxyrest oder ein H-Atom bedeutet,

a) wenn X den Chlorphenoxyrest bedeutet, hydrolysiert und den Chlorphenylrest abspaltet oder
b) wenn X ein H-Atom bedeutet, oxidiert.

Die Abspaltung des chlorierten Phenylrestes gemäß a) gelingt besonders gut, wenn man auf die Verbindungen III, die in einem organischen Lösungsmittelgemisch gelöst werden, ein Gemisch aus Nitrobenzaldoxim und Tetramethylguanidin während 0.5 - 2 Std. oder Tetrabutylammoniumfluorid 45 min. bei Raumtemperatur einwirken läßt.

Die Oxidation der Verbindungen III gelingt besonders gut bei Raumtemperatur mit Jod in organisch-wässrigen Lösungsmitteln.

Die Herstellung von Verbindungen der Formel I, worin $R^5$ für OH steht, kann in der Weise erfolgen, daß man eine Verbindung der Formel V

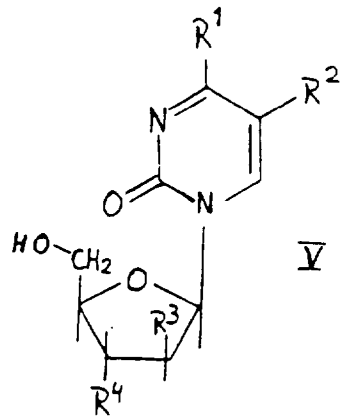

V

worin $R^1$ bis $R^4$ die angegebenen Bedeutungen haben, in an sich bekannter Weise selektiv in 5'-Stellung phosphoryliert oder daß man eine Verbindung der Formel Va

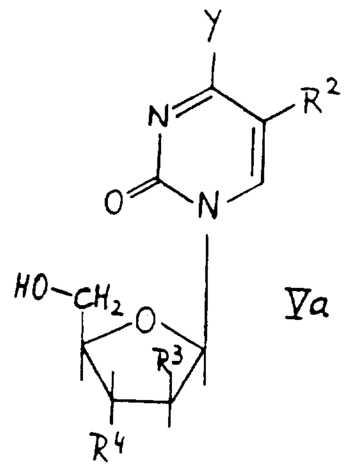

Va

worin $R^2$, $R^3$ und $R^4$ die angegebenen Bedeutungen haben und Y ein Chloratom oder den Rest:

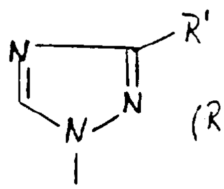

$(R' = H, NO_2)$

bedeutet,
mit einem Amin der Formel Vb

$H_2N\text{-}R''$ Vb

worin R" einen linearen oder verzweigten Alkylrest mit 12 bis 24 C-Atomen bedeutet, der bis zu 2 Doppelbindungen aufweist und durch einen aromatischen Rest substituiert sein kann, umsetzt und in den so erhaltenen Verbindungen gegebenenfalls vorhandene Acetylgruppen durch H-Atome ersetzt.

Die so erhaltenen Reaktionsprodukte können durch Chromatographie gereinigt werden.

Die als Ausgangsmaterial verwendeten Verbindungen der Formel III, bei denen X für Chlorphenoxy steht, lassen sich durch Kondensation von Verbindungen der Formel VI

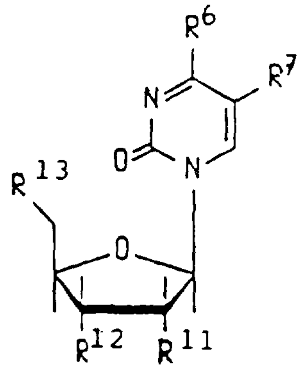

VI,

worin

$R^6$ und $R^7$, die angegebenen Bedeutungen haben, und $R^{11}$, $R^{12}$ und $R^{13}$ für H, Azido, Halogen, 4-Monomethoxytriphenylmethoxy, Acetyl oder 2-Chlorphenylphosphat stehen, wobei einer dieser Reste immer 2-Chlorphenylphosphat bedeutet, mit Verbindungen der Formel V

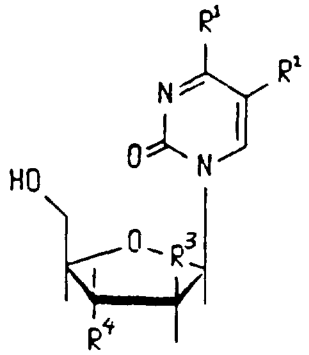

V,

worin $R^1$ bis $R^4$ die angegebene Bedeutungen haben, unter Zuhilfenahme von Kondensationsmitteln in an sich bekannter Weise herstellen (Makromol. Chem. 188, 1313 (1987)). Der 4-Monomethoxytriphenylmethylrest wird anschließend unter saueren Bedingungen, der Acetylrest unter alkalischen Bedingungen gegen Hydroxyl ausgetauscht.

Die als Ausgangsmaterial verwendeten Verbindungen der Formel III, bei denen X = H ist, lassen sich durch Kondensation einer Verbindung der Formel VI

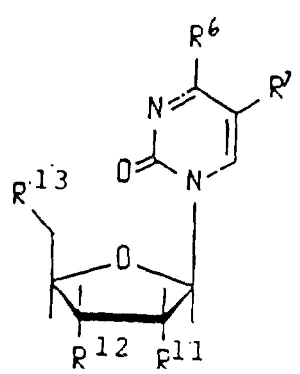

VI

worin
$R^6$ und $R^7$ die angegebenen Bedeutungen haben, $R^{11}$, $R^{12}$ und $R^{13}$ für H, Azido, Halogen, 4-Monomethoxytriphenylmethoxyl, Acetyl oder Hydrogenphosphonat stehen, wobei einer der Reste $R^{11}$, $R^{12}$, $R^{13}$ immer Hydrogenphosphonat bedeutet, mit einer Verbindung der Formel V in Gegenwart eines Säurechlorids und anschließender Oxidation in an sich bekannter Weise darstellen (Tetrahedron Lett. 27, 469 (1986)). Nach der Oxidation und chromatographischen Aufarbeitung wird der 4Monomethoxytriphylmethyl- und/oder Acetylrest gegen Hydroxyl ausgetauscht.

Die Verbindungen der Formel III können analog zur oben geschilderten Kondensation natürlich auch in der Weise hergestellt werden, daß der Phosphatrest via Verbindungen der Formel V zur Verfügung gestellt wird, wobei dann einer der Reste $R^{11}$, $R^{12}$, $R^{13}$ in Formel VI für OH steht.

Die für die Umsetzungen benötigten Ausgangsmaterialien sind bekannte Stoffe oder lassen sich in Analogie zu bekannten Verfahren herstellen (J.C.S. Perkin I 1171 (1982); Makromol. Chem. 187, 809 (1986); Tetrahedron Lett. 27, 2661 (1986)).

Die Überführung der therapeutisch wirksamen Nucleosidanaloga in amphiphile Dinucleosidphosphatanaloga bedingt ein stark verändertes pharmakokinetisches Verhalten, wodurch die zu applizierende Dosis im Vergleich zum jeweiligen Monomer erhöht werden kann ohne daß es gleichzeitig zu einer Verstärkung aller toxischen Nebeneffekte dieser hochpotenten Medikamente führt.

Der amphilphile Charakter der erfindungsgemäßen Nucleosidphosphatanaloga ermöglicht unterschiedliche Applikationsschemata. Die lipophile Nucleosidkomponente der Dinucleosidphosphatanaloga bewirkt, daß die Dimeren zusammen mit Matrixlipiden stabil in die Lipidmembran von Liposomen eingebaut werden. Die hydrophile Nucleosidkomponente ermöglicht, daß die Dimeren vermutlich über eine Micellenbildung auch wasserlöslich sind. Die lipophile Nucleosidkomponente begünstigt die Zellaufnahme der in Wasser gelösten amphiphilen Dinucleosidphosphatanaloga und schützt sie gleichzeitig vor einer zu schnellen enzymatischen Hydrolyse, so daß die gewünschte Depotwirkung der Dimeren nicht nur in einer Liposomendispersion sondern auch in wässrigen Lösungen erreicht wird. Die alternative Applikationsmöglichkeit der amphiphilen Dinucleosidphosphatanaloga hat den Vorteil, daß man nicht ausschließlich auf die Liposomentechnologie angewiesen ist, sie aber bei Bedarf nutzen kann, was wiederum bei nur wasserlöslichen Dinucleosidphosphaten nicht problemlos möglich ist.

Ein weiterer Vorteil der erfindungsgemäßen amphiphilen Dinucleosidphosphatanaloga liegt darin, daß sie zusammen mit einem oder mehreren anderen Wirkstoffen in unterschiedlichen Mengen in Liposomen eingebaut werden können, wobei synergistische Wirkungen resultieren. Mit diesen amphiphilen Dinucleosidphosphatanaloga und/oder in Zusammensetzung mit einem biologisch verträglichen Träger und/oder mit Mitteln, die die erfindungsgemäßen Verbindungen mindestens in einer oder mehreren der Zusammensetzungen enthalten, kann die Therapie von Krebserkrankungen und virusbedingten Infektionskrankheiten optimiert werden.

Für eine möglichst wirksame Applikation der erfindungsgemäßen Verbindungen werden weiterhin Zusammensetzungen verschiedener Art bereitgestellt. Allen diesen Zusammensetzungen ist gemeinsam, daß die erfindungsgemäßen Verbindungen in Verbindung mit einem organischen Träger stehen.

Eine bevorzugte Ausführungsform der Zusammensetzung sieht die Assoziation der erfindungsgemäßen Verbindungen in Form von uni-bis oligolamellaren Liposomen mit einem Durchmesser von maximal 0.4 µm vor. Die erfindungsgemäßen Verbindungen werden dabei in die Lipiddoppelschicht der Liposomen integriert. Zur Liposomenbildung können alle an sich bekannten Verfahren der Liposomendarstellung verwendet werden wie beispielsweise Ultraschall, Gelchromatographie, Detergenzanalyse. Die jeweils eingeführten lipophilen Reste beeinflussen maßgeblich die Größe und Stabilität der Liposomen, die sich aus den jeweiligen Dinucleosidphosphatanaloga zusammen mit weiteren Lipidkomponenten bilden.

Eine weitere Möglichkeit, die erfindungsgemäßen Verbindungen mit einem organischen Träger zu verbinden, ist der Einschluß der Verbindungen in ein biologisch verträgliches Nanopartikel. Als Nanopartikel werden organisch-chemische Polymere bezeichnet, denen bei der Polymerisation die erfindungsgemäßen Verbindungen zugesetzt werden, so daß sie mit einer gewissen Effizienz in das Nanopartikel eingeschlossen werden.

Die Zusammensetzung wird in einer bevorzugten Ausführungsform mit Komponenten ausgeführt, die sich in den zu therapierenden Zellen und/oder Organen spezifisch anreichern. Dabei kann beispielsweise die Zusammensetzung der Liposomen so gewählt werden, daß die Liposomen zusätzlich mit Molekülen wie z.B. Antikörper, geladene Lipide, mit hydrophilen Kopfgruppen modifizierte Lipide versehen werden, damit die Zusammensetzung sich bevorzugt in den zu therapierenden Zellen und/oder Organen anreichert. Eine solche Zusammensetzung mit spezifisch gegen Tumorzellen, virusinfizierten Zellen und/oder Organe gerichteten Molekülen erhöht die therapeutische Wirkung der Arzneimittel und reduziert gleichzeitig die Toxizität für nicht infiziertes Gewebe.

Die Zusammensetzungen können zu einem Mittel verarbeitet werden, das neben den erfindungsgemäßen Verbindungen und gegebenenfalls dem organischen Träger weiterhin übliche Träger- und/oder Verdünnungsmittel und/oder Hilfsstoffe enthält. Übliche Trägermittel sind beispielsweise Glucose, Dextrose, Albumine oder ähnliches, während als Verdünnungsmittel im wesentlichen physiologische Kochsalzlösungen oder eine 5%ige Glucoselösung dient. Weiterhin ist es üblich, die Lösungen mit geeigneten Reagenzien, beispielsweise Phosphaten, abzupuffern. Darüberhinaus können alle weiteren, für die Zubereitung von pharmazeutischen Mitteln üblichen Mittel hinzugesetzt werden, vorausgesetzt, sie greifen die Zusammensetzung aus dem organischen Träger und den erfindungsgemäßen Verbindungen nicht an. Das Mittel kann als Infusionslösung aber auch oral verabreicht werden.

Durch die Überführung in amphiphile Dinucleosidphosphatanaloga lassen sich aber nicht nur die enzymatische Hydrolysebeständigkeit erhöhen und die Applikationsformen deutlich erweitern, sondern überraschenderweise auch zytostatische und virusstatische Wirkungen optimieren.

Die amphiphile Dinucleosidphosphatanaloga lassen sich gegen maligne Erkrankungen der blutbildenden Zellen und andere Tumore einsetzen.

Durch die verbesserte zytostatische Wirkung treten sehr viel weniger gravierende Nebenwirkungen auf, es können

höhere Dosen der zytostatisch wirksamen erfindungsgemäßen Verbindungen eingesetzt werden und es kann in zeitlichen Intervallen therapiert werden.

Überraschenderweise zeigen die erfindungsgemäßen Dinucleosidphosphatanaloga auch virustatische Wirkungen, so daß sie in der Chemotherapie von virusbedingten Infektionen wie beispielweise Herpes und AIDS Erkrankung verwendbar sind.

Die folgenden Beispiele erläutern die Erfindung

Beispiel 1

Darstellung von 2'-Desoxythymidylyl-(3'-5')-$N^4$-palmitoyl-2',3'-didesoxycytidin.

a) Herstellung des Ausgangsmaterials

5.5 g (7.4 mmol) des Bariumsalzes von 5'-0-(4-Monomethoxy)triphenylmethyl-2'-desoxyribothymidin-3'-0-(2-chlorphenyl)phosphat werden zusammen mit 2.2 g (4.9 mmol) $N^4$-Palmitoyl-2',3'-didesoxycytidin in ca. 15 ml wasserfreiem Pyridin gelöst. Unter Feuchtigkeitsausschluß werden der Lösung zunächst 2.0 g (6.4 mmol) 2,4,6-Triisopropylbenzolsulfonsäurechlorid, wenige Minuten später 1.6 ml (19.7 mmol) N-Methylimidazol zugegeben. Anschließend wird das Reaktionsgemisch unter Feuchtigkeitsausschluß 40 min. bei Raumtemperatur geschüttelt und dann mit 5 ml Wasser versetzt. Die Lösung wird im Vakuum konzentriert, zweimal mit jeweils 50 ml Toluol abrotiert, in 50 ml Chlorofom aufgenommen und anschließend an einer Kieselgelsäule mit einem fünfstufigen Chloroform/Methanol-Gradienten fraktioniert. Das Produkt wird ab der 4. Gradientenstufe eluiert. Nach Trocknung des isolierten Eluats erhält man 3.7 g 5'-0-(4-Monomethoxy)-triphenyl-2'-desoxythymidylyl(2-chlorphenyl)-(3'-5')-$N^4$-palmitoyl-2',3'-didesoxycytidin als festen weißen Schaum.

b) Herstellung des Endprodukts

Der gemäß a) erhaltene Schaum wird zu einer Lösung von 3,0 g (10 mmol) Tetrabutylammoniumfluorid in 180 ml Tetrahydrofuran/Pyridin/Wasser; 8/1/1/; V/V/V gegeben und 45 min. bei Raumtemperatur gerührt. Die Lösung wird konzentriert, zweimal mit je 50 ml Toluol abrotiert und in 200 ml Ethylacetat aufgenommen. Es entsteht eine trübe Lösung, die jedoch keinen Niederschlag enthält. Diese wird mit 100 ml Wasser ausgeschüttelt. Aus der nun klaren organischen Phase fällt beim Ausschütteln mit gesättigter NaCl-Lösung das gewünschte Produkt als weißer, flockiger Niederschlag aus, der über eine Fritte abgetrennt wird. Der Vorgang wird wiederholt, bis kein Produkt mehr aus der Ethylacetatlösung ausfällt. Nach der Trocknung des Niederschlags am Ölpumpenvakuum erhält man 2.2 g 5'-0-(4-Monomethoxy)triphenylmethyl-2'-desoxythymidylyl-(3'-5')-$N^4$-palmitoyl-2',3'-didesoxycytidin als weißes, kristallines Pulver.

Zum Austausch der 4-Monomethoxytriphenylmethylgruppe gegen Hydroxyl wird das Produkt mit 50 ml Essigsäure/Wasser; 8/2; V/V versetzt und ca. 20 min. unter gelegentlichem Durchschütteln bei 50°C belassen. Die Lösung wird zum Öl konzentriert, das in 20 ml Chloroform/Methanol; 8/2; V/V aufgenommen und an einer Kieselgelsäule mit einem dreistufigen Chloroform/Methanol-Gradienten fraktioniert wird. Das Produkt wird ab der dritten Gradientenstufe eluiert. Nach Trocknung des isolierten Eluats erhält man 1.3 g 2'-Desoxythymidylyl-(3'-5')-$N^4$-palmitoyl-2',3'-didesoxycytidin als weißes feinkristallines Pulver (Zers.> 210°C), das auf der Kieselgeldünnschichtplatte im Laufmittelsystem Chloroform/Methanol; 6/4; V/V einen $R_f$-Wert von 0.63 aufweist.

Beispiel 2

Darstellung von $N^4$-Hexadecyl-2'-desoxycytidylyl-(3'-5')-2',3'-didesoxycytidin

a) Herstellung des Ausgangsmaterials

13.1 g (16.6 mmol) $N^4$-Hexadecyl-5'-0-(4-monomethoxy)triphenylmethyl-2'-desoxycytidin-3'-0-hydrogenphosphonat und 5 g (11.1 mmol) $N^4$-Palmitoyl-2',3'-didesoxycytidin werden in 30 ml wasserfreiem Pyridin mit 8 ml (64.5 mmol) Pivalinsäurechlorid unter Feuchtigkeitsausschluß versetzt. Die Lösung wird bei Raumtemperatur 10 min. gerührt, zum Sirup einrotiert, der dann zweimal mit 50 ml Toluol zum Öl abrotiert wird.

b) Herstellung des Endprodukts

Das gemäß a) erhaltene Öl wird mit 100 ml einer 0.2 M Jodlösung (Tetrahydrofuran/Pyridin/Wasser; 18/1/1; V/V/V) versetzt und 40 min. bei Raumtemperatur belassen. Nach dieser Oxidation wird der Reaktionsansatz mit einem Gemisch aus 350 ml Chloroform und 350 ml 2 %iger wässriger $NaHSO_3$-Lösung ausgeschüttelt. Die organische Phase wird

zweimal mit 100 ml gesättigter NaCl-Lösung gewaschen, über $Na_2SO_4$ getrocknet, im Vakuum bis auf 100 ml konzentriert, die dann an einer Kieselgelsäule in dreistufigem Chloroform/Methanol-Gradienten fraktioniert werden. Das gewünschte Produkt wird ab der dritten Gradientenstufe eluiert. Das isolierte Eluat wird im Vacuum zum Sirup konzentriert.

Zum Austausch der 4-Monomethoxy)triphenylmethylgruppe gegen Hydroxyl wird der Sirup mit 50 ml Essigsäure/Wasser; 4/1; V/V 12 h bei Raumtemperatur behandelt und anschließend an einer Kieselgelsäule gereinigt. Das hierbei isolierte Eluat wird wiederum zum Sirup konzentriert, in 50 ml Methanol, das mit Ammoniak gesättigt ist, aufgenommen und verschlossen 12 h bei Raumtemperatur belasssen. Nach einer erneuten chromatographischen Reinigung an Kieselgel und Aufarbeitung des isolierten Eluats erhält man $N^4$-Hexadecyl-2'-desoxycytidylyl-(3'-5')-2',3'-didesoxycytidin als weißes feinkristallines Pulver, das sich oberhalb von 180°C zersetzt und im Laufmittelsystem Chloroform/Methanol; 7/3; V/V einen $R_f$-Wert von 0.39 aufweist.

Beispiel 3

Darstellung von $N^4$-Hexadecyl-2'-desoxycytidylyl-(3'-5')-5-ethyl-2'-desoxyuridin

a) Herstellung des Ausgangsmaterials

5.1 g (5.4 mmol) des Natriumsalzes von $N^4$-Hexadecyl-5'-0-(4-monomethoxy)triphenylmethyl-2'-desoxycytidin-3'-0-(2-chlorphenyl)phosphat werden zusammen mit 1.4 g (5.4 mmol) 5-Ethyl-2'-desoxyuridin in Analogie zu Beispiel 1 kondensiert. Das Kondensationsgemisch wird an einer Kieselgelsäule mit Chloroform chromatographisch gereinigt.

b) Herstellung des Endprodukts

Der gemäß a) erhaltene Schaum wird 90 min. bei Raumtemperatur mit 20 ml einer Lösung behandelt, die folgende Zusammensetzung aufweist. In 80 ml Chloroform/Methanol; 9/1;V/V werden 10 g Nitrobenzaldoxim und 5 ml Tetramethylguanidin gelöst. Nach der Reaktion wird das Gemisch an einer Kieselgelsäule mit Chloroform/Methanol-Gradienten fraktioniert. Das isolierte Eluat wird im Vacuum zum Sirup konzentriert. Zum Austausch der 4-Monomethoxytriphenylmethylgruppe gegen Hydroxyl wird der Sirup mit 50 ml Essigsäure/Wasser; 4/1 12 h bei Raumtemperatur behandelt. Die Lösung wird am Rotationsverdampfer zum Sirup konzentriert, der in 5 ml Methanol aufgenommen wird. Durch Eintropfen in 100 ml Ether fällt das gewünschte Produkt aus. Der Niederschlag wird abgenutscht, getrocknet, in 25 ml Chloroform aufgenommen und wiederum an einer Kieselgelsäule mit Chloroform/Methanol fraktioniert. Das hierbei isolierte Produkt wird in 5 ml Wasser aufgenommen und an einer Sephadex G-10 Säule mit Wasser chromatographiert. Das Produkt enthaltende Eluat wird lyophilisiert, wobei $N^4$-Hexadecyl-2'-desoxycytidylyl-(3'-5')-5-ethyl-2'-desoxyuridin erhalten wird, das im Laufmittelsystem Chloroform/Methanol; 1/1; V/V einen $R_f$-Wert von 0.54 und einen Zersetzungspunkt > 169°C aufweist.

Beispiel 4

Darstellung von $N^4$-Hexadecyl-5-methyl-3'-azido-2',3'-didesoxycytidin (im folgenden mit $hxd^4m^5$AzddCyd abgekürzt).

a) Darstellung der Ausgangsverbindung 4-(1,2,4-Triazol-1-yl)-5-methyl-1-(β-D-3'-azido-5'-O-acetyl-2',3'-didesoxyribofuranosyl-2(1H)-pyrimidinon.

13.4 g (50 mmol) käufliches 2'-Azido-2',3'-didesoxythymidin (AZT) werden in 100 ml wasserfreiem Pyridin mit 23.5 ml (250 mmol) Essigsäureanhydrid 5 h bei Raumtemperatur gerührt, dann mit 20 ml Methanol versetzt und erneut 15 min. gerührt. Die Lösung wird am Rotationsverdampfer zu Sirup konzentriert, der zur Entfernung von Pyridin mit Toluol abrotiert wird. Der Rückstand wird in 160 ml wasserfreiem Acetonitril aufgenommen und dann zu der, im folgenden beschriebenen Reaktionslösung zugetropft.

31.1 g (450 mmol) 1,2,4-Triazol werden in 260 ml wasserfreiem Acetonitril suspendiert, mit 8.8 ml (96 mmol) Phosphorylchlorid versetzt und im Eisbad auf ca. 4°C gekühlt. Unter kräftigem Rühren läßt man in das gekühlte Reaktionsgemisch innerhalb von 1 h 60 ml (431 mmol) Triethylamin und anschließend die obige Lösung des AZT-Derivats innerhalb von 1 h zutropfen. Dann wird das Eisbad entfernt und das Reaktionsgemisch weitere 45 min. gerührt. Der Niederschlag wird abgenutscht, zweimal mit je 50 ml Acetonitril gewaschen und verworfen. Das Filtrat wird mit 45 ml Trietylamin und 12 ml Wasser versetzt, nach 10 min. im Vacuum auf ca. 50 ml konzentriert und dann mit 600 ml Chloroform, 500 ml 2%-iger wässrige Natriumhydrogencarbonatlösung aufgeschüttelt. Die wässrige Phase wird zweimal mit je 100 ml Chloroform extrahiert, die vereinigten Chloroformphasen werden am Rotationsverdampfer zum Sirup konzentriert, der mit ca. 300 ml Ethanol in der Wärme gelöst wird. Beim Erkalten kristallisiert das gewünschte Produkt aus und wird

nochmals aus 15o ml Ethanol umkristallisiert. Nach dem Trocknen im Vacuum werden 14.7 g (41 mmol) weiße Kristalle (Fp. 143-144°C) erhalten.

b) Herstellung des Endproduktes

Zu dem Syntheseprodukt aus a), das in 120 ml Dioxan gelöst ist, werden innerhalb von 1 h 13.5 g (55 mmol) Hexadecylamin, gelöst in 120 ml Ethanol, bei Raumtemperatur zugetropft. Der Reaktionsansatz wird 1.5 h gerührt, dann im Vacuum zum Sirup konzentriert und in 500 ml Methanol aufgenommen, die mit Ammoniak bei Raumtemperatur gesättigt wurden. Der gut verschlossene Reaktionsansatz wird nach 12 h Stehen bei Raumtemperatur im Vacuum zum Sirup konzentriert, der anschließend an einer Kieselgelsäule mit Chloroform/Methanol-Gradienten chromatographisch gereinigt wird. Die Fraktionen des gewünschten Produkts werden im Vacuum vom Lösungsmittel befreit. Hierbei erhält man 16.0 g $N^4$-Hexadecyl-5-methyl-3'-azido-2,3'-didesoxycytidin als Sirup, das auf der Kieselgelplatte im System Chloroform/Methanol; 9/1; V/V einen $R_F$-Wert von 0.52 aufweist. In der FD-Massenspektroskopie wird für den Molekülpeak + H der Wert 491.1 gefunden und somit die berechnete Molekülmasse von 490,7 bestätigt.

Beispiel 5

Darstellung von $N^4$-Palmitoyl-2',3'-dideoxycytidin-5'-phosphat

Zu einer bei 0°C gerührten Mischung aus 1.09 ml (12 mmol) Phosphorylchlorid und 20 ml Trimethylphosphat werden nach und nach 2.7 g (6 mmol) $N^4$-Palmitoyl-2',3'-dideoxycytidin zugegeben. Der Ansatz wird weitere 2 h in der Kälte gerührt und dann mit 0.5 N NaOH unter Kühlung langsam neutralisiert. Der ausgefallene Niederschlag wird abzentrifugiert, in Chloroform/Methanol; 7/3; V/V aufgenommen, über Natriumsulfat filtriert und im Vakuum vom Lösungsmittel befreit. Der hierbei erhaltene Rückstand wird mehrmals aus Ethanol kristallisiert. Hierbei werden 2.5 g $N^4$-Palmitoyl-2',3'-dideoxycytidin-5'-phosphat erhalten, die auf der Kieselgelplatte im System Chloroform/Methanol; 1/1; V/V einen $R_f$-Wert von 0.35 aufweisen.

Beispiel 6

Darstellung von $N^4$-Palmitoyl-2'-desoxyribocytidylyl-(3'-5')-3'-azido-2',3'-didesoxythymidin im folgenden mit $N^4$-palmitoyldC-AZT abgekürzt.

a) Herstellung des Ausgangsmaterials

8.0 g (10 mmol) $N^4$-Palmitoyl-5'-O-(4-monomethoxy)triphenylmethyl-2'-desoxyribocytidin-3'-O-hydrogenphosphat und 2,7 g (10 mmol) 3'-Azido-2',3'-didesoxythymidin wurden in 30 ml wasserfreiem Pyridin mit 6,2 ml (50 mmol) Pivalinsäurechlorid unter Feuchtigkeitsausschluß versetzt. Die Lösung wurde bei Raumtemperatur 10 Min. gerührt, zum Sirup einrotiert, der dann mit 20 ml Toluol abrotiert wurde.

b) Herstellung des Endproduktes

Der gemäß a) erhaltene Rückstand wurde mit 100 ml einer 0,2 M Jodlösung (Tetrahydrofuran/Pyridin/Wasser; 18/1/1; V/V/V) versetzt und 40 Min bei Raumtemperatur belassen. Nach dieser Oxidation wurde der Reaktionsansatz mit einem Gemisch aus 350 ml Chloroform und 350 ml 2 %iger wässriger $NaHSO_3$-Lösung ausgeschüttelt. Die organische Phase wurde über $Na_2SO_4$ getrocknet, im Vakuum bis auf 50 ml Chloroform konzentriert und an einer Kieselgelsäule in Chloroform/ Methanol-Gradienten fraktioniert. Die Fraktionen des gewünschten Produkts, die mit Chloroform/Methanol; 9/1; V/V die Säule verließen, wurden vereinigt, zur Trockne konzentriert und anschließend aus Methanol kristallisiert.

Zum Austausch der Triphenylmethoxygruppen gegen Hydroxylgruppen wurde der erhaltene Niederschlag in 200 ml Essigsäure/Wasser; 4/1; V/V bei Raumtemperatur 12 Std. gerührt. Die Lösung wurde dann im Vakuum zur Trockne einrotiert. Der Rückstand wurde in wenig Methanol aufgenommen und durch Eintropfen in Ether ausgefällt.

Der Niederschlag wurde abgesaugt, im Vakuum getrocknet, in Chloroform aufgenommen und erneut an einer Kieselgelsäule mit Chloroform/Methanol-Gradienten fraktioniert. Die Fraktionen des gewünschten Produkts, die mit Chloroform/Methanol; 7/3; V/V die Säule verließen, wurden zum Sirup konzentriert, der in wenig Wasser aufgenommen an einer Sephadex G-15 Säule chromatographisch gereinigt wurde. Die Produktfraktionen wurden konzentriert, und der Rückstand aus Ethanol kristallisiert. Hierbei wurden 4,6 g (5,8 mmol) $N^4$-Palmitoyl-2'-desoxyribocytidylyl-(3'--5')-3'-azido-2',3'-didesoxythymidin erhalten, das auf der Kieselgelplatte im Laufmittelsystem Chloroform/Methanol; 1/1; V/V einen $R_f$-Wert von 0,54 aufwies.

Beispiel 7

Darstellung von $N^4$-Hexadecyl-2'-desoxyribocytidylyl-(3'--5')-3'-azido-2',3'-didesoxythymidin im folgenden mit $N^4$-HexadecyldC-AZT abgekürzt.

a) Herstellung des Ausgangsmaterials

9,4 g (10 mmol) des Natriumsalzes von $N^4$-Hexadecyl-5'-O-(4-monomethoxy)triphenylmethyl-2'-desoxyribocytidin-3'-O-(2 chlorphenyl)phosphat und 2,7 g (10 mmol) 3'-Azido-2',3'-didesoxythymidin wurden in 20 ml wasserfreiem Pyridin gelöst. Unter Feuchtigkeitsausschluß wurden der Lösung zunächst 3,6 g (12 mmol) 2,4,6-Triisopropylbenzolsulfonsäurechlorid, wenige Minuten später 2,8 ml (36 mmol) N-Methylimidazol zugegeben. Anschließend wurde das Reaktionsgemisch unter Feuchtigkeitsausschluß 40 Min. bei Raumtemperatur gerührt und dann mit 5 ml Wasser versetzt. Die Lösung wurde im Vakuum konzentriert, zweimal mit jeweils 50 ml Toluol abrotiert, in 30 ml Chloroform aufgenommen und anschließend an einer Kieselgelsäule mit Chloroform fraktioniert. Die Fraktionen des gewünschten Produkts wurden im Vakuum zum Sirup konzentriert.

b) Herstellung des Endprodukts

Der gemäß a) erhaltene Sirup wurde 90 Min. bei Raumtemperatur mit 60 ml einer Lösung behandelt, die folgende Zusammensetzung aufwies: In 80 ml Chloroform/Methanol; 9/1; V/V wurden 10 g Nitrobenzaldoxim und 5 ml Tetramethylguanidin gelöst. Anschließend wurde das Reaktionsgemisch an einer Kieselgelsäule mit Chloroform/Methanol-Gradienten fraktioniert und weiter wie im Beispiel 6 aufgearbeitet. Nach der Kristallisation aus Ethanol wurden 2,6 g (3.3 mmol) $N^4$-Hexadecyl-2'-desoxyribocytidylyl-(3'--5') -3'-azido-2',3'-didesoxythymidin erhalten, das im Laufmittelsystem Chloroform/Methanol; 1/1; V/V einen $R_f$-Wert von 0,56 aufwies.

Beispiel 8

Herstellung einer Zusammensetzung, enthaltend einen organischen Träger, eine der erfindungsgemäßen amphiphile Dinucleosidphosphatanaloga, Cholesterol und ein Antioxidans.

1. Zusammensetzung der Liposomen

Liposomen mit 10 mg der erfindungsgemäßen Verbindung pro ml.

100 ml Liposomen enthalten: 4.0 g Phosphatidylcholin, 0.4 g Cholesterol, 0.02 g α-DL-Tocopherol, 1.0 g Dinucleosidphosphatanaloga dispergiert in 0.9 % Kochsalzlösung, die, wenn nötig, zum Beispiel mit Phosphatpuffer auf einen gewünschten pH-Wert eingestellt ist. Anstelle der gegebenenfalls gepufferten Kochsalzlösung kann auch ein 67 mM physiologischer Phosphatpuffer oder eine 5 %ige Glucoselösung verwendet werden.

2. Herstellung

Herstellung von 100 ml Liposomendispersion, die 10 mg der erfindungsgemäßen Verbindung pro ml enthalten.

Die Lipide (4.0 g Phosphatidylcholin, 0.4 g Cholesterol, 0.02 g α-DL-Tocopherol und 1.0 g amphiphile Dinucleosidphosphatanaloga werden mit einer geeigneten Menge Methylenchlorid/Methanol; 1/1; V/V (100 bis 500 ml) in einem 500 ml Rundkolben gelöst. Die organischen Lösungsmittel werden bei 40°C in einem Rotationsverdampfer entfernt. Die entstandene, lösungsmittelfreie Lipid/Wirkstoff Mischung wird durch Zugabe einer entsprechenden Menge von 0.9% Kochsalzlösung, die zum Beispiel mit 10 mM Phosphatpuffer abgepuffert sein kann, in 100 ml solubilisiert. Anstelle der gegebenenfalls gepufferten Kochsalzlösung kann auch ein 67 mM physiologischer Phosphatpuffer oder eine 5%ige Glucoselösung verwendet werden. Die entstehenden, sogenannten multilamellaren Liposomen können mit einem geeigneten Verfahren, z.B. Ultrabeschallung, Hochdruckfiltration, Detergensdialyse, Mikroemulgation oder anderen geeigneten Methoden, weiterverarbeitet werden.

Beispiel 9

Darstellung der Liposomenpräparate durch Ultraschall

Für die Herstellung von Liposomendispersionen werden pro ml Chloroform/Methanol; 1/1; V/V jeweils 100 mg Soja-Phosphatidylcholin, 10 mg Cholesterol, 1 mg α-DL-Tocopherol, 7 mg $N^2$-Palmitoyl-$N^6$-succinoyl-L-lysin und 2 bis 12 mg der erfindungsgemäßen amphiphilen Dinucleosidphosphatanaloga gelöst. 0.6 bis 2.4 ml dieser Lipidstammlösung

werden in einem Reagenzglas durch Verblasen mit Luft in einen Lipidfilm überführt, der anschließend ca. 1 h bei 50°c im Vakuum getrocknet wird. Der Lipidfilm wird mit 3 ml 10 mM PBS (0.9 % NaCl und 10 mM $NaH_2PO_4$, pH 7.3) versetzt und mit Hilfe einer Mikrospitze eines Desintegrators 30 min. mit 40 Watt beschallt. Hierbei bildet sich eine opaleszente Liposomendispersion.

Tabelle 1

Physikalisch-chemische Eigenschaften von Liposomen (10 mg $N^4$-HexadecyldC-AZT/ml)

| Herstellungsart | Hydrodynamischer Durchmesser (nm)[a] | Homogenität[b] | Einbaurate[c] in % |
|---|---|---|---|
| Hochdruckfiltration | 170 | 0.52 | 95 ± 6 |
| Detergensdialyse | 22 | 0.40 | 98 ± 4 |
| Ultraschall | 86 | 0.56 | 100 |

a) Hydrodynamischer Durchmesser und

b) Homogenität wurden mittels Laser-Lichtstreuung bestimmt. Homogenität: 0.2 bis 0.5 homogene Doppelschicht Liposomen; > 0.5 heterogene Populationen von unterschiedlich grossen Liposomen. Referenzmessungen mit homogenen Latexkugeln ergaben Werte von 0.2 bis 0.5 Homogenität, was einer Gauss'schen Normalverteilung mit kleiner Standardabweichung entspricht.

c) Die Einbaurate der erfindungsgemäßen Verbindung $N^4$-HexadecyldC-AZT in die Liposomen-Doppelschicht wurde UV-spektroskopisch bestimmt.

Beispiel 10

Darstellung der Immunoliposomen

1.2 nmol eines Antikörpers werden als Lyophilisat mit 50 µl des Liposomenpräparats aus Beispiel 7 und 7 mg (27 µmol) N(3-Dimethylaminopropyl)-N'-ethylcarbodiimid X HCL (EDC) versetzt und durch Zugabe von 30 µl PBS (pH 1) auf pH 4 eingestellt. Im einstündigen Abstand werden noch zweimal jeweils 7 mg EDC zugegeben. Nach ca. 5 h Rühren

bei Raumtemperatur wird der Reaktionsansatz auf eine Ultrogel ACA 22-Säule aufgetragen und mit PBS (pH 7.4) fraktioniert. Fraktionen deren Absorptionsverhältnisse mit den Werten des eingesetzten Liposomenpräparats übereinstimmen, werden vereinigt und zum Zelltargeting verwendet.

Beispiel 11

Anti-retrovirale Wirkung von $N^4$-Hexadecyldc-AZT Liposomen

Experimentelle Bedingungen

Am Tag 0 wurden intravenös 4 bis 5 x $10^4$ Viruseinheiten (plaque forming units, siehe Ruprecht et al., Nature 323, 466, 1986) des Rauscher Leukämievirus in Balb/C Mäuse injiziert.

Die in Tabelle 2 angegebenen Dosen wurden an den Tagen 1,6,11,16 intraperitioneal oder intravenös appliziert.

Tabelle 2

Bedingungen und Ergebnisse der intraperitonealen (ip) oder intravenösen (iv) Therapie an den Tagen 1,6,11,16 nach der Virusinfektion mit Azidothymidin in Phosphatpuffer (A) oder der erfindungsgemäßen Verbindung $N^4$-HexadecyldC-AZT als Liposomenpräparat (B).

| Wirkstoff | Dosierung[a] in mg/kg Körpergewicht | Therapie | Milzgewicht in mg ± SA[b] | I[c] in % |
|---|---|---|---|---|
| A | 380 | iv | 1,99 ± 0,35 | - 5,9 |
| B | 380 | iv | 1,19 ± 0,19 | 48,4 |
| A | 380 | ip | 1,31 ± 0,09 | 31,6 |
| B | 407 | ip | 1,26 ± 0,51 | 37,5 |
| A | 760 | ip | 1,66 ± 0,26 | 9,7 |
| B | 814 | ip | 0,94 ± 0,25 | 60,2 |
| A | 1140 | ip | 1,59 ± 0,27 | 13,7 |
| B | 1221 | ip | 0,32 ± 0,06 | 93,9 |
| Kontrollen | | | | |
| infiziert | -- | ip | 1,79 ± 0,20 | 0 |
| uninfiziert | -- | ip | 0,15 ± 0,03 | -- |
| infiziert | -- | iv | 1,86 ± 0,27 | 0 |
| uninfiziert | -- | iv | 0,14 ± 0,05 | -- |

a) Dosis bezogen auf AZT als aktiver Bestandteil des erfindungsgemäßen $N^4$-HexadecyldC-AZT.

b) SA, Standardabweichung

c) Inhibition der Milzvergrößerung berechnet nach der oben angegebenen Formel

Auswertung

Am Tag 20 nach Virusinfektion wurden die Mäuse nach Anästhesie getötet und sofort die Milz entnommen. Das Milzgewicht wurde bestimmt und die prozentuale Inhibition der Milzvergrößerung (Splenomegalie) nach folgender Formel berechnet:

$$I(\%) = [\, 1 - x/(c-n)] \times 100,$$

wobei

| | |
|---|---|
| I(%) = | Inhibition der Splenomegalie in % |
| x = | mittleres Milzgewicht behandelter infizierter Mäuse |
| c = | mittleres Milzgewicht unbehandelter infizierter Mäuse |
| n = | mittleres Milzgewicht unbehandelter uninfizierter Mäuse. |

Beispiel 12

Die virusstatische Wirkung der neuen amphiphilen Dinucleosidphosphatanaloga läßt sich in folgender Versuchsanordnung zeigen:
Mäuse-Embryo-Zellkulturen werden mit Herpes simplex Viren (HSV-1 oder HSV-2) infiziert. Zu diesen Zellkulturen gibt man die amphiphilen Dinucleosidphosphatanaloga, die im Medium in Konzentrationen von 500 µl/ml, 50 µl/ml und 5 µl/ml gelöst sind. Nach 3 Tagen Inkubationszeit zeigt sich an der Reduktion der Plaquezahl die virustatische Wirkung. Hierbei zeigen die neuen Verbindungen bessere Wirkungen als beispielsweise das bekannte Virustatikum 5-Ethyl-2'-desoxyuridin.

## Patentansprüche

**1.** Amphiphile Nucleosidphosphatanaloga der Formel I

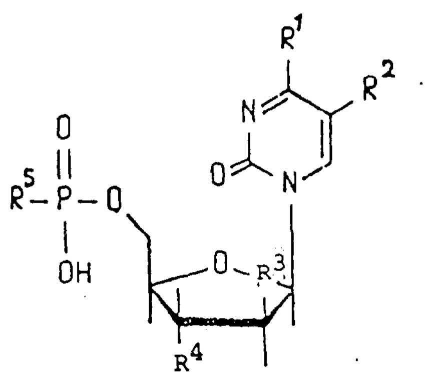

I,

worin

$R^1$ eine Hydroxyl-, Amino-, acylierte oder alkylierte oder durch Polyoxyethylen substituierte Aminogruppe bedeutet, deren Acyl- oder Alkylrest linear oder verzweigt ist, 1 bis 24 C-Atome und bis zu 2 Doppelbindungen aufweist und durch einen aromatischen Rest substituiert sein kann;

$R^2$ für H, F, einen 2-Bromvinylrest oder einen linearen oder verzweigten $C_1$-$C_{24}$-Alkylrest steht;

$R^3$ und $R^4$ gleich oder verschieden sind und für H, Hydroxyl, Halogen und Azido stehen;

$R^5$ einen Nucleosidrest der Formel II

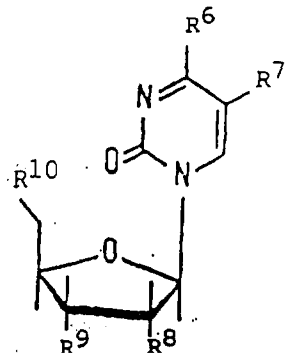

II,

bedeutet, worin,

- $R^6$ eine Hydroxyl-, Amino-, acylierte oder alkylierte Aminogruppe bedeutet, deren Acyl- oder Alkylrest linear oder verzweigt ist, 1 bis 24 C-Atome und bis zu 2 Doppelbindungen aufweist und durch einen aromatischen Rest substituiert sein kann, wobei
- $R^6$ und $R^1$ verschieden sein sollen und einer der Reste für Acylamino- oder Alkylaminoreste mit 12 bis 24-C-Atomen steht;
- $R^7$ für H, F, einen 2-Bromvinylrest oder einen linearen oder verzweigten $C_1$ bis $C_{24}$-Alkylrest steht;
- einer der Reste $R^8$ bis $R^{10}$ ein Sauerstoffatom bedeutet, das die Brücke zum Nucleotid der Formel I bildet, die beiden verbleibenden Reste gleich oder verschieden sind und für H, Hydroxyl, Halogen oder Azido stehen;
- $R^5$ auch OH bedeuten kann, wenn $R^1$ und/oder $R^2$ lipophile Reste darstellen; und
- $R^1$ und/oder $R^2$ lipophile Reste und $R^6$ und/oder $R^7$ hydrophile Reste darstellen oder vice versa oder die Reste $R^1$, $R^2$, $R^6$ und $R^7$ so gewählt werden, daß sie gemeinsam einem Dinucleosidphosphatanalog amphiphilen Charakter verleihen; sowie die entsprechenden Salze der sauren Formen dieser Verbindungen.

**2.** Verbindungen nach Anspruch 1, worin $R^6$ eine Oktadecyl- oder Docosyl-Aminogruppe oder eine Palmitoyl-, Oleoyl- oder Behenoyl-Aminogruppe bedeutet und $R^7$ und $R^8$ für H stehen.

**3.** Verbindungen nach einem der vorhergehenden Ansprüche, worin

$R^1$ eine Amino- oder Hydroxylgruppe,

$R^2$ ein H-Atom oder Methyl bedeuten,

$R^3$, $R^4$, $R^9$ und $R^{10}$ gleich oder verschieden sind und für H, Hydroxyl, Azid, Halogen stehen und einer der Reste $R^8$, $R^9$ oder $R^{10}$ ein Sauerstoffatom darstellt, das die Brücke zum Nucleotid der Formel I bildet.

**4.** Verbindungen nach einem der vorhergehenden Ansprüche, worin $R^3$ und/oder $R^4$ für H, Hydroxyl und/oder Azido stehen.

**5.** Verbindungen nach Anspruch 2, worin

$R^1$, $R^4$ für Hydroxyl,

$R^2$ für Ethyl oder Fluor,

$R^3$ für H stehen und

$R^9$ oder $R^{10}$ ein Sauerstoffatom bedeuten, das die Brücke zum Nucleotid der Formel I darstellt.

**6.** Verbindungen nach einem der vorhergehenden Ansprüche, worin

$R^6$ für Hydroxyl,

$R^7$ für Methyl,

$R^2$ für H oder Methyl und $R^8$ für H,

$R^3$ und/oder $R^4$ für H, OH, Azido stehen und einer der Reste $R^9$, $R^{10}$ ein Sauerstoffatom bedeutet, das die Brücke zum Nucleotid der Formel I bildet und der andere Rest für H oder OH steht.

**7.** Verbindungen nach einem der vorhergehenden Ansprüche, worin

$R^1$ eine Palmitoyl-, Oleoyl- oder Behenoyl-Aminogruppe bedeutet und

$R^3$ und/oder $R^4$ für H oder OH stehen.

**8.** Verbindungen nach einem der vorhergehenden Ansprüche, worin

$R^1$ eine Oktadecyl- oder Docosyl-Aminogruppe und

$R^3$ und $R^4$ Hydroxyl bedeuten.

**9.** Mittel zur Behandlung von Infektionskrankheiten und/oder Krebserkrankungen, enthaltend mindestens eine Verbindung nach einem der vorhergehenden Ansprüche in einem pharmazeutisch verträglichen Träger oder Verdünnungsmittel, gegebenenfalls in Verbindung mit einem pharmazeutisch verträglichen Formulierungsmittel, oder eingebaut in Liposomen.

**10.** Verfahren zur Herstellung der Verbindungen der Formel I der Ansprüche 1 bis 8, dadurch gekennzeichnet, daß man Verbindungen der Formel III

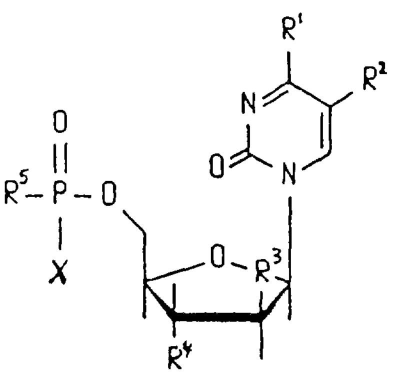

III,

worin $R^1$ bis $R^5$ die angegebenen Bedeutungen haben, $R^5$ aber nicht für OH steht, und X den Chlorphenoxyrest oder ein H-Atom bedeutet,

a) wenn X den Chlorphenoxyrest bedeutet, hydrolysiert und den Chlorphenylrest abspaltet oder

b) wenn X ein H-Atom bedeutet, oxidiert.

**11.** Verfahren zur Herstellung von Verbindungen der formel I der Ansprüche 1 bis 8, worin $R^5$ für OH steht, dadurch gekennzeichnet, daß man eine Verbindung der Formel V

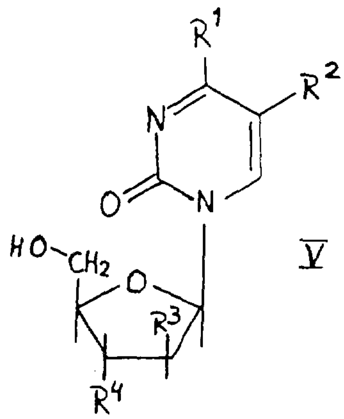

V

worin $R^1$ bis $R^4$ die angegebenen Bedeutungen haben, in an sich bekannter Weise selektiv in 5'-Stellung phosphoryliert oder daß man eine Verbindung der Formel Va

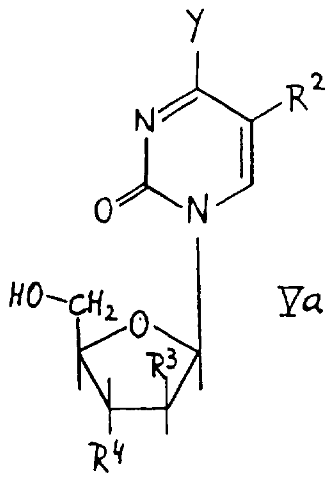

Va

worin $R^2$, $R^3$ und $R^4$ die in den Ansprüchen 1 bis 9 angegebenen Bedeutungen haben und Y ein Chloratom oder den Rest:

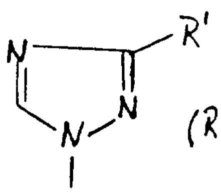

($R' = H, NO_2$)

bedeutet,
mit einem Amin der Formel Vb

$H_2N\text{-}R''$ Vb

worin R" einen linearen oder verzweigten Alkylrest mit 12 bis 24 C-Atomen bedeutet, der bis zu 2 Doppelbindungen aufweist und durch einen aromatischen Rest substituiert sein kann, umsetzt und in den so erhaltenen Verbindungen gegebenenfalls vorhandene Acetylgruppe durch H-Atome ersetzt.

## Claims

**1.** Amphiphilic nucleoside phosphate analogues of formula I

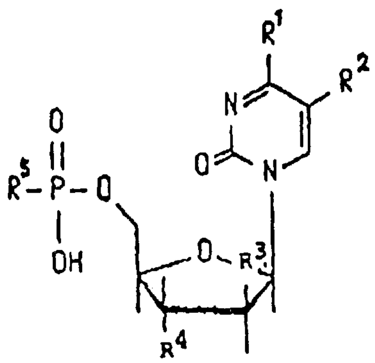

I,

wherein

$R^1$ denotes a hydroxyl or amino group or an acylated or alkylated or polyoxyethylene-substituted amino group, the acyl or alkyl group of which is straight-chained or branched, has 1 to 24 carbon atoms and up to 2 double bonds and may be substituted by an aromatic group;

$R^2$ denotes H, F, a 2-bromovinyl group or a straight-chained or branched $C_{1-24}$-alkyl group;

$R^3$ and $R^4$ are identical or different and denote H, hydroxyl, halogen and azido;

$R^5$ denotes a nucleoside group of formula II

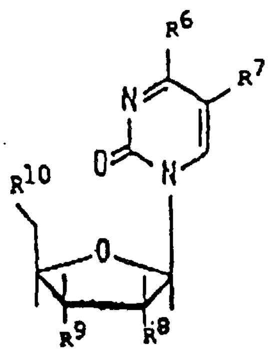

II,

wherein

- $R^6$ denotes a hydroxyl or amino group or an acylated or alkylated amino group, the acyl or alkyl group of which is straight-chained or branched, contains 1 to 24 carbon atoms and up to 2 double bonds and may be substituted by an aromatic group, whilst
- $R^6$ and $R^1$ shall be different and one of the groups denotes acylamino or alkylamino groups having 12 to 24 carbon atoms;
- $R^7$ denotes H, F, a 2-bromovinyl group or a straight-chained or branched $C_{1-24}$-alkyl group;
- one of the groups $R^8$ to $R^{10}$ denotes an oxygen atom which forms the bridge to the nucleotide of formula I, the two remaining groups are identical or different and denote H, hydroxyl, halogen or azido;
- $R^5$ may also denote OH if $R^1$ and/or $R^2$ represent lipophilic groups; and
- $R^1$ and/or $R^2$ denote lipophilic groups and $R^6$ and/or $R^7$ denote hydrophilic groups or vice versa or the groups $R^1$, $R^2$, $R^6$ and $R^7$ are selected so that, together, they impart an amphiphilic nature to a dinucleoside phosphate analogue; and the corresponding salts of the acid forms of these compounds.

**2.** Compounds according to claim 1, wherein $R^6$ denotes an octadecyl- or docosyl-amino group or a palmitoyl-, oleoyl- or behenoyl-amino group and $R^7$ and $R^8$ denote H.

**3.** Compounds according to one of the preceding claims, wherein

$R^1$ denotes an amino or hydroxyl group,

$R^2$ denotes an H-atom or methyl,

$R^3$, $R^4$, $R^9$ and $R^{10}$ are identical or different and represent H, hydroxyl, azide or halogen and one of the groups $R^8$, $R^9$ or $R^{10}$ denotes an oxygen atom which forms the bridge to the nucleotide of formula I.

**4.** Compounds according to one of the preceding claims, wherein $R^3$ and/or $R^4$ denote H, hydroxyl and/or azido.

**5.** Compounds according to claim 2, wherein

$R^1$, $R^4$ denote hydroxyl,

$R^2$ denotes ethyl or fluorine,

$R^3$ denotes H and

$R^9$ or $R^{10}$ denote an oxygen atom which forms the bridge to the nucleotide of formula I.

**6.** Compounds according to one of the preceding claims, wherein

$R^6$ denotes hydroxyl,

$R^7$ denotes methyl,

$R^2$ denotes H or methyl and $R^8$ denotes H,

$R^3$ and/or $R^4$ denotes H, OH or azido and one of the groups $R^9$, $R^{10}$ denotes an oxygen atom which forms the bridge to the nucleotide of formula I and the other group denotes H or OH.

**7.** Compounds according to one of the preceding claims, wherein

$R^1$ denotes a palmitoyl-, oleoyl- or behenoyl-amino group and

$R^3$ and/or $R^4$ denotes H or OH.

**8.** Compounds according to one of the preceding claims, wherein

$R^1$ denotes an octadecyl- or docosyl-amino group and

$R^3$ and $R^4$ denote hydroxyl.

**9.** Composition for treating infectious diseases and/or cancers, containing at least one compound according to one of the preceding claims in a pharmaceutically acceptable carrier or diluent, optionally in conjunction with a pharmaceutically acceptable formulating agent, or incorporated in liposomes.

**10.** Process for preparing the compounds of formula I according to claims 1 to 8, characterised in that compounds of formula III

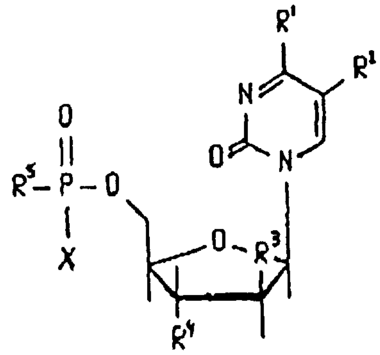

III,

wherein $R^1$ to $R^5$ are as hereinbefore defined but $R^5$ does not denote OH, and X denotes the chlorophenoxy group or an H-atom,

a) are hydrolysed, if X denotes the chlorophenoxy group, and the chlorophenyl group is cleaved or

b) are oxidised, if X denotes an H-atom.

**11.** Process for preparing compounds of formula I according to claims 1 to 8, wherein $R^5$ denotes OH, characterised in that a compound of formula V

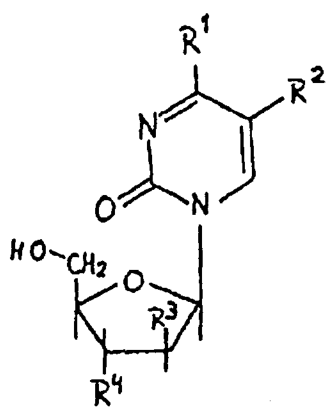

V

wherein $R^1$ to $R^4$ are as hereinbefore defined, is selectively phosphorylated in the 5'-position in a manner known _per se_ or a compound of formula Va

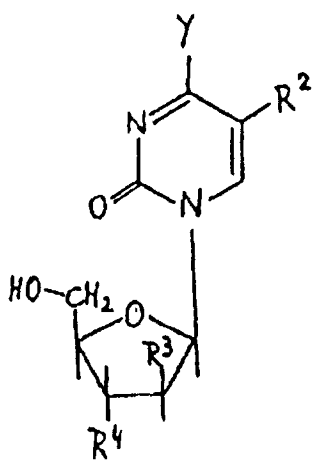

Va

wherein $R^2$, $R^3$ and $R^4$ have the meanings given in claims 1 to 9 and Y denotes a chlorine atom or the group:

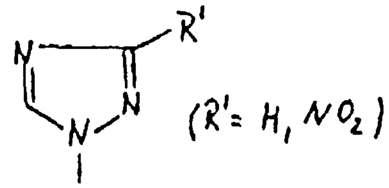

is reacted with an amine of formula Vb

$$H_2N\text{-}R'' \qquad Vb$$

wherein R" denotes a straight-chained or branched $C_{12-24}$-alkyl group which has up to 2 double bonds and may be substituted by an aromatic group, and any acetyl which may be present in the resulting compounds is replaced by H-atoms.

## Revendications

**1.** Composés analogues des phosphates nucléosides amphiphiles de formule I

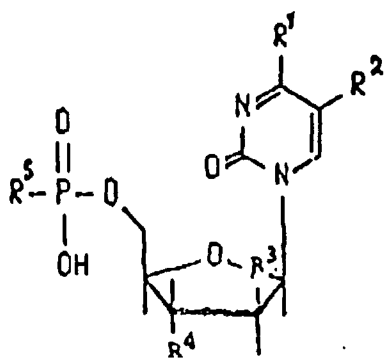

I,

dans laquelle

$R^1$ désigne un groupe hydroxyle, amino, amino acylé, alkylé ou substitué par du polyoxyéthylène dont le radical acyle ou alkyle est linéaire ou ramifié, comporte de 1 à 24 atomes de C et jusqu'à deux liaisons doubles et peut être substitué par un radical aromatique,

$R^2$ désigne H, F, un radical 2-bromovinyle ou un radical alkyle en $C_1$ à $C_{24}$ linéaire ou ramifié,

$R^3$ et $R^4$ sont identiques ou différents et représentent H, hydroxyle, halogène et azido,

$R^5$ désigne un radical nucléoside de formule II

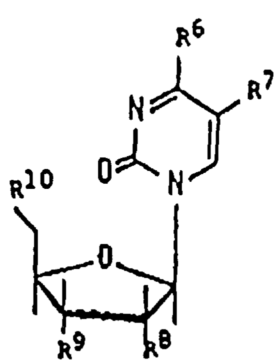

II,

dans laquelle

- $R^6$ désigne un groupe hydroxyle, amino, amino acylé ou alkylé dont le radical acyle ou alkyle est linéaire ou ramifié, comporte de 1 à 24 atomes de C et jusqu'à deux liaisons doubles et peut être substitué par un

radical aromatique ;

- $R^6$ et $R^1$ devant être différents et l'un de ces radicaux désignant des radicaux acylamino ou alkylamino ayant de 12 à 24 atomes de C,
- $R^7$ désignant H, F, un radical 2-bromovinyle ou un radical alkyle en $C_1$ à $C_{24}$ linéaire ou ramifié,
- l'un des radicaux $R^8$ à $R^{10}$ désignant un atome d'oxygène qui constitue le pont vers le nucléotide de formule I, les deux radicaux restants étant identiques ou différents et désignant H, hydroxyle, halogène et azido,
- $R^5$ pouvant également désigner OH, lorsque $R^1$ et/ou $R^2$ représentent des radicaux lipophiles, et
- $R^1$ et/ou $R^2$ représentant des radicaux lipophiles et $R^6$ et/ou $R^7$ représentant des radicaux hydrophiles, ou inversement, ou les radicaux $R^1$, $R^2$, $R^6$ et $R^7$ étant choisis de façon qu'ensemble, ils confèrent à un analogue de phosphate dinucléoside un caractère amphiphile, ainsi que les sels correspondant des formes acides de ces composés.

**2.** Composés selon la revendication 1, dans lesquels $R^6$ désigne un groupe octadécylamino ou docosylamino ou un groupe palmitoylamino, oléoylamino ou béhénoylamino et $R^7$ et $R^8$ désignent H.

**3.** Composés selon l'une des revendications précédentes, dans lesquels

$R^1$ désigne un groupe amino ou hydroxyle,

$R^2$ est H ou méthyle,

$R^3$, $R^4$, $R^9$ et $R^{10}$ sont identiques ou différents et désignent H, hydroxyle, azide, halogène et l'un des radicaux $R^8$, $R^9$ ou $R^{10}$ représente un atome d'oxygène qui constitue le pont vers le nucléotide de formule I.

**4.** Composés selon l'une des revendications précédentes, dans lesquels $R^3$ et/ou $R^4$ désignent H, hydroxyle et/ou azido.

**5.** Composés selon la revendication 2, dans lesquels

$R^1$, $R^4$ désignent hydroxyle,

$R^2$ désigne éthyle ou fluor,

$R^3$ désigne H, et

$R^9$ ou $R^{10}$ désignent un atome d'oxygène qui constitue le pont vers le nucléotide de formule I.

**6.** Composés selon l'une des revendications précédentes, dans lesquels

$R^6$ désigne hydroxyle,

$R^7$ désigne méthyle,

$R^2$ désigne H ou méthyle et $R^8$ désigne H,

$R^3$ et/ou $R^4$ désignent H, OH, azido, et
l'un des radicaux $R^9$, $R^{10}$ représente un atome d'oxygène qui constitue le pont vers le nucléotide de formule I et l'autre radical désigne H ou OH.

**7.** Composés selon l'une des revendications précédentes, dans lesquels

$R^1$ désigne un groupe palmitoylamino, oléoylamino ou béhénoylamino, et

$R^3$ et/ou $R^4$ désignent H ou OH.

8. Composés selon l'une des revendications précédentes, dans lesquels

$R^1$ désigne un groupe octadécylamino ou docosylamino, et

$R^3$ et $R^4$ désignent hydroxyle.

9. Composition pour le traitement de maladies infectieuses et/ou d'affections cancéreuses, contenant au moins un composé selon l'une des revendications précédentes dans un support ou diluant pharmaceutiquement acceptable, éventuellement on association avec un agent de formulation pharmaceutiquement acceptable, ou incorporé dans des liposomes.

10. Procédé de préparation des composés de formule I selon les revendications 1 à 8, caractérisé par le fait que l'on traite des composés de formule III

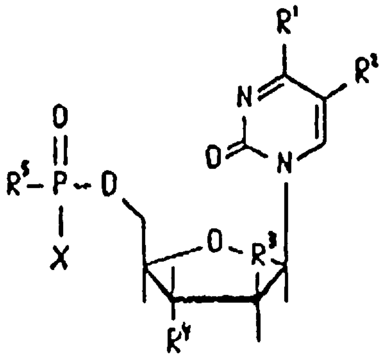

III,

dans laquelle $R^1$ à $R^5$ ont les significations indiquées, $R^5$ ne désignant cependant pas OH, et X désigne le radical chlorophénoxy ou H,

a) lorsque X désigne le reste chlorophénoxy, par hydrolyse et séparation du radical chlorophényle, ou

b) lorsque X est H, par oxydation.

11. Procédé de préparation des composés de formule I selon les revendications 1 à 8, où $R^5$ désigne OH, caractérisé par le fait que l'on phosphorylise sélectivement en position 5', de manière connue en soi, un composé de formule V

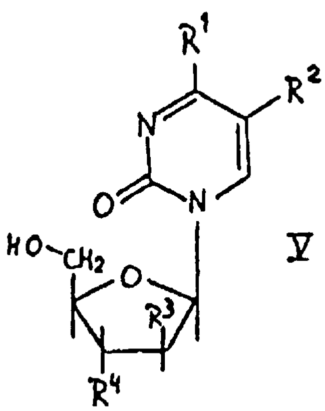

V .

dans laquelle $R^1$ à $R^4$ ont les significations indiquées, ou on fait réagir un composé de formule Va

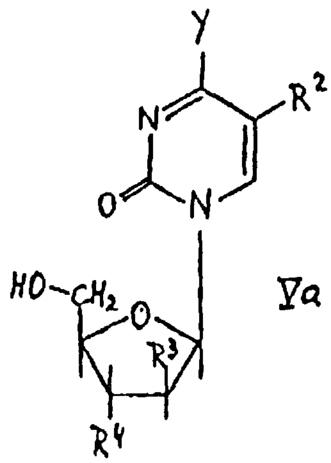

Va

dans laquelle $R^2$, $R^3$ et $R^4$ ont les significations indiquées dans les revenications 1 à 9 et Y désigne un atome de chlore ou le radical :

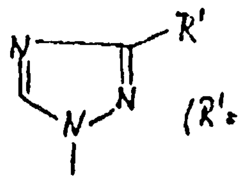

($R'$ = H, $NO_2$)

avec une amine de formule Vb

$$H_2N\text{-}R'' \quad Vb$$

dans laquelle R" désigne un radical alkyle linéaire ou ramifié ayant de 12 à 24 atomes de C qui représente jusqu'à deux liaisons doubles et peut être substitué par un radical aromatique, et que l'on remplace le groupe acétyle éventuellement présent dans les composés ainsi obtenus par H.